(19)

Europäisches
Patentamt
European
Patent Office
Office européen
des brevets

(11) **EP 1 957 663 B1**

(12) **EUROPEAN PATENT SPECIFICATION**

(45) Date of publication and mention
of the grant of the patent:
**02.09.2009 Bulletin 2009/36**

(21) Application number: **06851909.9**

(22) Date of filing: **03.11.2006**

(51) Int Cl.:
*C12Q 1/02* (2006.01)     *G01N 33/53* (2006.01)

(86) International application number:
**PCT/US2006/042984**

(87) International publication number:
**WO 2008/054394 (08.05.2008 Gazette 2008/19)**

(54) **METHOD AND APPARATUS FOR ENHANCED DETECTION OF TOXIC AGENTS**

VERFAHREN UND VORRICHTUNG FÜR DEN VERBESSERTEN NACHWEIS VON GIFTSTOFFEN

PROCÉDÉ ET DISPOSITIF PERMETTANT UNE MEILLEURE DÉTECTION DES AGENTS
TOXIQUES

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **10.11.2005 US 271643**

(43) Date of publication of application:
**20.08.2008 Bulletin 2008/34**

(73) Proprietor: **UT-BATTELLE, LLC
Oak Ridge, TN 37831 (US)**

(72) Inventors:
• **GREENBAUM, Elias
Knoxville, TN 37931 (US)**
• **RODRIGUEZ, Miguel, Jr.
Oak Ridge, TN 37830 (US)**
• **WU, Jie, Jayne
Knoxville, TN 37920 (US)**
• **QI, Hairong
Knoxville, TN 37932 (US)**

(74) Representative: **Eisenführ, Speiser & Partner
Patentanwälte Rechtsanwälte
Postfach 10 60 78
28060 Bremen (DE)**

(56) References cited:
**US-B2- 6 569 384**

• YEH S-R ET AL: **"Assembly of ordered colloidal
aggregates by electric-field-induced fluid flow"
NATURE, NATURE PUBLISHING GROUP,
LONDON, GB, vol. 386, no. 6620, 6 March 1997
(1997-03-06), pages 57-59, XP002201553 ISSN:
0028-0836**
• BHATT KETAN H ET AL: **"An AC electrokinetic
technique for collection and concentration of
particles and cells on patterned electrodes."
LANGMUIR : THE ACS JOURNAL OF SURFACES
AND COLLOIDS 5 JUL 2005, vol. 21, no. 14, 5 July
2005 (2005-07-05), pages 6603-6612,
XP002475757 ISSN: 0743-7463 cited in the
application**

## Description

FIELD OF THE INVENTION

The present invention relates to biosensors for detecting chemical, biological and/or radiological contaminants, or their precursors, in water or air.

BACKGROUND OF THE INVENTION

There is an increased awareness of the possibility of attacks on metropolitan areas using chemical, biological and radiological warfare agents. Researchers at the Oak Ridge National Laboratory (ORNL) have developed a biosensor system to detect toxic agents in primary-source drinking water, such as disclosed in U.S. Pat. No. 6,569,384 to Greenbaum et al. through the analysis of fluorescence induction curves. U.S. Pat. No. 6,569,384 to Greenbaum et al. discloses a water quality sensor for detecting the presence of at least one chemical or biological warfare agent. The sensor includes a cell, apparatus for introducing water into the cell and discharging water from the cell adapted for analyzing photosynthetic activity of naturally-occurring, free-living, indigenous photosynthetic organisms in water; a fluorometer for measuring photosynthetic activity of naturally-occurring, free-living, indigenous photosynthetic organisms drawn into the cell. An electronics package analyzes raw data from the fluorometer and emits a signal indicating the presence of at least one chemical or biological warfare agent in the water. Although the water quality sensor disclosed in U.S. Pat. No. 6,569,384 to Greenbaum et al. provides highly useful devices, it would be desirable to improve the speed and sensitivity of the device.

SUMMARY

A method of biosensor-based detection of toxins comprises the steps of concentrating a plurality photosynthetic organisms in a fluid to be analyzed into a concentrated region using biased AC electro-osmosis, and obtaining a measured photosynthetic activity of the photosynthetic organisms in the concentrated region, wherein chemical, biological or radiological agents reduce a nominal photosynthetic activity of the photosynthetic organisms. The presence of at least one of the agents, or precursors thereof, in the fluid is determined based on the measured photosynthetic activity. The plurality of photosynthetic organisms can be naturally-occurring, free-living, indigenous organisms in the fluid, such as native algae in water. The DC bias for the biased ACEO is generally from 1 to 10 volts.

Biased ACEO according to the invention provides significant fluorescent signal enhancement due to resulting enhanced concentration of the photosynthetic organisms. Enhanced concentration of the photosynthetic organisms significantly improving the sensitivity and peed of devices and methods according to the invention as compared to previous related devices and methods.

The photosynthetic activity can comprise chlorophyll fluorescence induction. A lab-on-a-chip system can be used for the concentrating step. The fluid can be drawn from a source of primary-source drinking water. In this embodiment, the method can further comprise the step of refreshing a supply of photosynthetic organisms by drawing a fresh supply of drinking water and repeating the method.

In a preferred embodiment of the invention, advanced signal processing algorithms are used utilized to detect the existence of the toxic agents. In such an embodiment, the determining step can further comprises the steps of providing at least one time-dependent control signal generated by a biosensor in the fluid medium, obtaining a time-dependent biosensor signal from the biosensor in the fluid medium to be monitored or analyzed for the presence of one or more of the chemical, biological or radiological agents; processing the time-dependent biosensor signal to obtain a plurality of feature vectors using at least one of amplitude statistics and a time-frequency analysis, and determining the presence of at least one of the agents, or precursors thereof, from the feature vectors based on reference to the control signal. The time-frequency analysis can comprise wavelet coefficient analysis. In one embodiment, both amplitude statistics and time-frequency analysis can be used in the processing step.

The method can further comprising the step of identifying which agents are present in the fluid. A linear discriminant method can be used for the identifying step. The linear discriminant method can comprise support vector machine (SVM) classification.

A water quality analyzer comprises a biased AC electro-osmosis (ACEO) cell for receiving a fluid to be analyzed having a plurality photosynthetic organisms therein and concentrating the plurality photosynthetic organisms into at least one concentrated region. A photodetector obtains a measured photosynthetic activity of the plurality of photosynthetic organisms in the concentrated region, wherein chemical, biological or radiological agents reduce a nominal photosynthetic activity of the photosynthetic organisms. An electronics package analyzes the measured photosynthetic activity to indicate a presence of the chemical, biological or radiological agents in the fluid. The ACEO cell can comprise a lab-on-a-chip device. The lab-on-a-chip device can include at least one electronic device on the chip. A structure can also be provided for communicating the measured photosynthetic activity or the measured photosynthetic activity after analysis by the electronics package to one or more remote sites.

BRIEF DESCRIPTION OF THE DRAWINGS

A fuller understanding of the present invention and the features and benefits thereof will be obtained upon review of the following detailed description together with the accompanying drawings, in which:

Fig. 1 is a schematic view showing an exemplary AC electro-osmosis (ACEO)-based water quality analyzing system 100, according to an embodiment of the present invention.

Fig. 2 shows an exemplary AC electro-osmosis (ACEO) cell based on planar interdigitated electrodes, according to an embodiment of the present invention.

Fig. 3 shows a schematic of preferred embodiment of the ACEO cell-based system along with related interface devices, according to an embodiment of the present invention. The ACEO trapping cell is embodied as a lab-on-a-chip device in this preferred embodiment.

Fig. 4 is an exemplary fluorescence induction curve.

Fig. 5 compares the average amplitude statistics generated from the control and exposure signals of each toxic agents class.

Fig. 6 shows a 3-D plot showing three amplitude statistics features (mean, standard deviation, and skewness). It can be seen from Fig. 6 that data from different classes of toxic agents cluster at different positions within the 3D feature space that can be separated relatively easily.

Fig. 7 shows some examples of some exemplary mother wavelets, including the Daubechies wavelet, the Coiflet wavelet, the Harr wavelet, the Symmlet wavelet, the Meyer wavelet and the Battle Lemarie wavelet.

Fig. 8 shows a filter bank implementation of a discrete wavelet transform.

Fig. 9 shows an example of a fluorescence induction curve exposed to 10 mM KCN and the corresponding 3-level wavelet decomposition.

Fig. 10(a)-(d) show fluorescence induction curves exposed to four classes of toxic agents and the corresponding wavelet transforms of these signals.

DETAILED DESCRIPTION

As noted in the background, conventional aquatic biosensors monitor the quality of primary-source drinking water by analyzing the fluorescence signal signature from healthy algae during photosynthesis. Fluorescence emitted by healthy algae differs from that emitted by algae exposed to a toxic agent. Relatively simple algorithms based on PSII efficiency are generally used to characterize the signature of the fluorescence signal. However, a major problem encountered in toxic detection is that microalgae are present at a low concentration so that reliable detection is often not possible.

Sensitivity and selectivity are critical for real-time detection of toxins. For selectivity, a tissue based detection system is preferably utilized which uses naturally-occurring aquatic photosynthetic tissue as the sensing material (microalgae) for detection of antagonists in the water. For sensitivity, the microbial concentration scheme uses biased AC electro-osmosis (ACEO), which is newly discovered and presently at the forefront of electrokinetic microfluidic research. ACEO rapidly enriches local microalgal concentration by expediting their diffusion via microfluidic convection to certain areas, thus allowing their concentration. Concentrated biosensors improves the resulting signal level, analogous to the function of an amplifier in an electronic circuit. Fluorescence techniques are then preferably used to assess the metabolic health of the biosensors.

A water quality analyzer for real-time detection according to the invention comprises a biased AC electro-osmosis (ACEO) cell for receiving a fluid to be analyzed having a plurality photosynthetic organisms therein, and concentrating the plurality photosynthetic organisms into at least one concentrated region. A photodetector, such as luminometer or fluorometer, is provided for obtaining a measured photosynthetic activity of the plurality of photosynthetic organisms in the concentrated region, wherein chemical, biological or radiological agents reduce a nominal photosynthetic activity of the photosynthetic organisms. An electronics package analyzes the measured photosynthetic activity to indicate a presence of the chemical, biological or radiological agents in the fluid. Specific agents can also be identified. For example, using characteristic fluorescence induction curves for a given library of agents, specific agents can be identified. Assuming the agent is a certain minimum concentration, the concentration of the agent may also be determined.

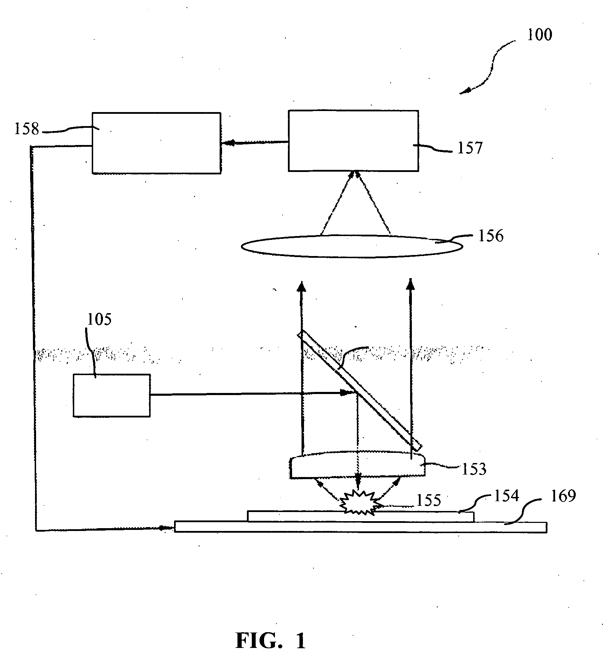

Figure 1 is a schematic view showing an exemplary AC electro-osmosis (ACEO)-based water quality analyzing system 100, according to an embodiment of the present invention. The system 100 comprises a light source 105 for emitting light beam, a beam splitter 152 for redirecting the emitted light beam to a focusing lens 153. Focusing lens 153 focuses the light beam after reflection from beam splitter onto biased AC electro-osmosis (ACEO) trapping cells 154. When the photosynthetic organisms are bioluminescent, light source 105, beam splitter 152 and focusing lens 153 are not required. ACEO cell 154 receives a fluid to be analyzed having a plurality photosynthetic organisms therein and concentrates the plurality photosynthetic organisms into at least one concentrated region therein. Light is directed towards the concentrated regions in ACEO cell 154 thus exciting the photosynthetic organisms on the ACEO cell 154 to produce fluorescence 155. A focusing lens 156 is provided for focusing the fluorescence on a photodetector 157 which detects the fluorescence. A computing device 158 receives the measured fluorescence signal detected by the photodetector 157.

The ACEO cell 154 is preferably disposed on a platform 169. The platform is generally movable under the control of computing device 158. Computing device is generally loaded with algorithms to analyze the fluorescence data as well as to control the movement of the platform 169.

There are generally two types of ACEO particle traps comprising the configurations of planar interdigitated electrodes and parallel plate electrodes. Planar interdigitated electrodes are generally preferred for the present invention. Parallel plates typically generate uniform electric field normal to the electrode surface. In this configuration, tangential electric fields are generated by asymmetric electrode patterns on the plates. Patterned electrodes are preferably provided as disclosed in a paper by Bhatt, et al (Bhatt, K.H., Grego, S., Velev, O.D. 2005; An AC electrokinetic technique for collection and concentration of particles and cells on patterned electrodes. Langmuir, 21(14):6603 -6612). Electrodes face each other, in the form of parallel plates. The electric field is generated by Bhatt et al. using a pure AC signal. No DC bias is disclosed or suggested by Bhatt. The nonuniform electric field gradients are realized through patterning one electrode with non-electrically conductive materials; such as photoresist, or silicon dioxide. Particles accumulate at the center of corrals where the flow velocity is relatively low.

In contrast to Bhatt et al., the invention preferably uses an AC signal together with a DC bias, referred to herein as "biased ACEO" to bias the electrodes. Biased ACEO takes advantage of different electrode polarizations which are not possible based on the AC only ACEO disclosed in Bhatt et al.. Significantly, the inventive biased ACEO can produce a distinct line of particles, thus heavily concentrating the particles. For a plate separation of about 0.5 mm, the DC bias is generally in a range from 1 to 10 volts, while the AC signal has less than a 20 volt peak to peak amplitude and frequency of from 50 Hz to 1 MHz. Preferably, the voltage range is within 10 Vrms for DC and AC components combined.

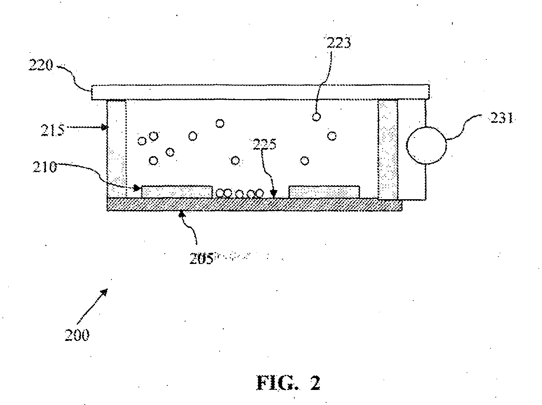

An exemplary ACEO cell 200 based on planar interdigitated electrodes according to the present invention is shown in Fig. 2. A substrate support 205 having at least semiconducting electrical conductivity is provided to provide a bottom electrode; such as an n- or p-doped silicon wafer 205. A patterned layer of an electrically insulating material 210, such as silicon dioxide, is disposed on the substrate support 205. An electrically insulating spacing material 215 surrounds and seals the periphery of cell 200 to allow a liquid to be analyzed to be held within the cell. Any substrate 205 can generally be used, as long as an electrically conductive layer is placed on top of the substrate 205 and that electrically conductive layer is exposed to the fluid.

The exposed patterned electrode is generally realized either by applying another layer of insulting layer or by etching the conductive layer. Fluid stagnation forms at the center of the electrode patterns 225 where the photosynthetic organisms 223 get concentrated.

The nominal height of spacing material is generally on the order of hundreds of microns. An optically transparent top electrode 220 in the wavelength range of interest, such as an indium-tin-oxide coated grass layer, is disposed on spacing material to complete the cell enclosure. A plurality of photosynthetic organisms 223 are disposed inside cell 200. A power supply 231 which provides an AC signal riding on a DC bias applies an electrical signal across top electrode 220 and bottom electrode 205. Although not shown, separate power supplies for the AC signal and the DC bias can be used.

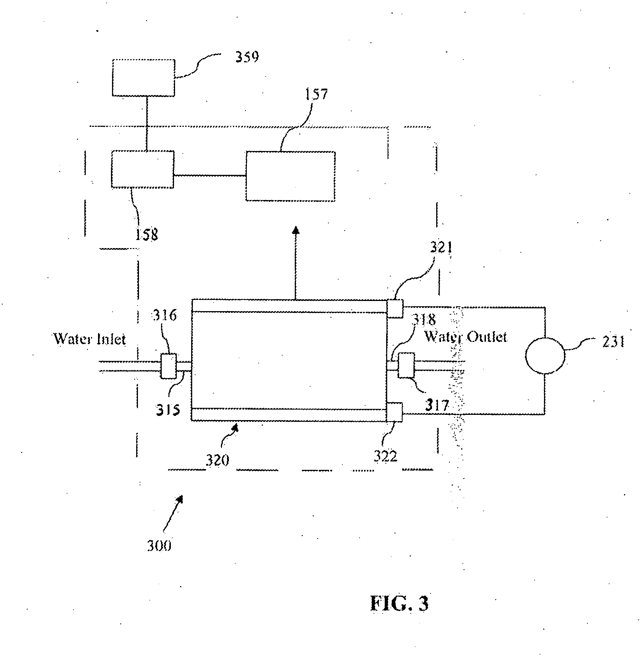

Figure 3 shows a preferred embodiment of the ACEO cell-based system 300 along with related interface devices. ACEO trapping cell is embodied as a lab-on-a-chip device 320 in this preferred embodiment. The lab-on-a-chip provides rapid and sensitive biological and clinical analyses on a single, miniaturized device. Lab-on-a-chip devices are a subset of MEMS devices.

There are several advantages to the lab-on-a-chip embodiment along with microalgae sensors, including rapid detection time from electrokinetic enrichment, specificity as a result of using microalgae as sensors, and simplicity since the detection components are located on a single platform. The lab-on-a-chip technology also provides portability and reduced analysis time. The reduction of sample and reactant volume increases the efficiency and reduces the costs associated with analytical chemistry and analytical biochemistry. Additionally, microfabrication provides the possibility of mass production. As shown by the dashed line in Fig. 3 which indicates a single chip, lab-on-a-chip device 320, as well as electronic components such as photodetector 157 and computing device 158 can be fabricated on the same chip.

Electrical contact to lab-on-a-chip device 320 is provided by external electrodes 321 and 322. An AC power supply 231 provides the required bias to realize ACEO and forces the biosensors into at least one concentrated region. In one embodiment, a battery (not shown) provides the power for the various components of system 300.

Lab-on-a-chip device 320 includes a water inlet 315 and water outlet 318. Water is generally driven into inlet 315 by a small internal pump 316 that are commonly used for lab-on-a-chip devices. Water is also drawn out from outlet 318 by small internal pump 317. Periodically, new samples of water are preferably analyzed by system 100 using a "batch mode" rather than a "continuous mode". One unique aspect of the invention is that unlike conventional sensing devices, this sensor material is external to the detecting instrument and can be continuously refreshed. Such systems thus may be used as continuous rapid-warning sentinels for detection of chemical warfare agents in sunlight-exposed drinking water supplies.

System 100 includes a structure for communicating measured and/or analyzed data to one or more remote sites 359. The remote sites can be military or civilian sites. For example, in military applications, military personnel could use the invention in the field. In civilian applications, water utilities personnel may use the invention at the intake points for water reservoirs located far from their water treatment plant or distribution facility. For example structure for communicating 359 can comprise Wi-Fi™ card 150. Card 150 connects to a local area network (LAN) when near a network access point. The connection is made by radio frequency signals. If the local area network is connected to the Internet, the Wi-Fi device can have Internet access as well. Alternatively, structure for communicating 359 can comprise an RS 232 interface or equivalent for serial binary data interconnection of the measured data and or analyzed data.

In one embodiment, raw data received from the photodetector 157 is transmitted by Wi-Fi™ 359 through a LAN to a remote cite where a processor/computer is located (not shown). The processor/computer obtains the measured photosynthetic activity of the photosynthetic organisms in the concentrated region, and applies an algorithm to determining a presence of chemical, biological or radiological agents, or precursors thereof, based on the measured photosynthetic activity, and in a preferred embodiment can also determine the specific agent as well as its concentration.

In one embodiment of the invention, water samples can be stored and time stamped. In a preferred embodiment of the invention, an operator can detect at what time and date the samples were collected to permit further analysis to comply with regulatory mandates (e.g. federal, state, city, industrial, etc.) in the event of the detection of a severe ' toxic agent. Microchannels on the lab-on-a-chip 320 can be used for this purpose. In a preferred embodiment of the invention, the system allows the operator to remove designated chips/cartridges containing the microchannels storing the water collected from such an event. By activating certain internal pumps or opening certain water lines and deactivating other pumps or closing certain water lines, the system could be designed to direct the water from the main set of microchannels to the ones used for storing purposes.

In order to detect the existence of toxic agents, the traditional method is to measure the so-called "efficiency of PSII (photosystem II) photochemistry";

$$PSII \text{ efficiency} = \frac{F_{max} - F_s}{F_{max}}$$

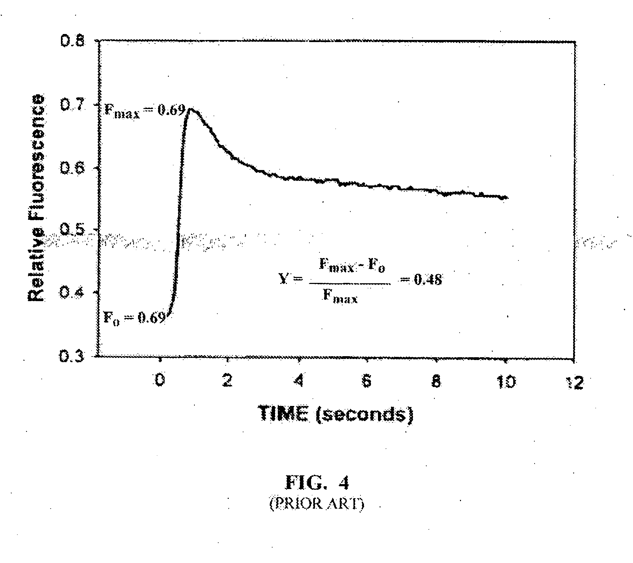

where $F_s$ is the value at the stable time and $F_{max}$ is the maximum value of the fluorescence induction curve, as shown in Fig. 4. The PSII efficiency represents a simple induction curve-based calculation of the fluorescence signal "signature", and significant deviations thereof indicate the potential presence of a toxic agent in the water.

Although PSII efficiency is generally effective in detecting the presence of toxic agents, it fails in some cases due to the non-significant photochemical yield presented by certain toxic agents. Moreover, it cannot generally classify between different agents or the same agent with different concentrations. In addition, using this parameter it can take as long as 60 minutes to arrive at a decision regarding detection of a contamination event. The classification of different agents with a shorter response time is of profound importance, such as to reduce response time to a contamination event. With the knowledge of the type of toxic agent, appropriate medicine and rescue strategies can be used in time to save lives as well as counter the terrorist attacks.

In a preferred embodiment, advanced signal processing algorithms are utilized to detect the existence of toxic agents. This advanced analysis methodology comprises the steps of providing at least one time-dependent control signal generated by a biosensor in a liquid (e.g. water) or a gas (e.g. air), and obtaining a time-dependent biosensor signal from the biosensor in a gas or liquid medium to be monitored or analyzed for the presence of one or more toxins selected from chemical, biological or radiological agents. The time-dependent biosensor signal is processed to obtain a plurality of feature vectors using at least one of amplitude statistics and a time-frequency analysis. At least one parameter relating to the toxicity of the gas or liquid medium is then determined from the feature vectors based on reference to the control signal provided. As used herein, the phrase "features vector" is defined as (i) summation based statistical measures as described below (amplitude statistics) and (ii) coefficients (e.g. wavelet coefficients), or statistical parameters derived from the coefficients (e.g. wavelet coefficient standard deviation) generated by application of a time-frequency analysis to the time-dependent sensor signal.

A first new algorithm comprises high-order statistical analysis (referred to herein as "amplitude statistics") of the light signal in the time domain. As used herein, the phrase "amplitude statistics" is defined as summation based statistical measures derived from a plurality of (N) time points in the signal curve, such as first order (mean), second order (standard deviation), third order (skewness), and fourth order (kurtosis). PS II efficiency, such as disclosed in U.S. Pat. No. 6,569,384 to Greenbaum et al., is thus clearly not amplitude statistics since the measurement therein is based on the simple difference between discrete points being the maximum value of the fluorescence induction curve ($F_{max}$) and the fluorescence value at the stable time ($F_s$).

Amplitude statistics can capture more dynamic features of the signal than PSII efficiency, including how fast the signal approaches maximum and minimum, how far samples are from the mean value, and how symmetric the signal appears. These features are generally required in the detection and identification regarding the existence of different toxic agents.

A first new algorithm comprises wavelet analysis of the light signal in the time-frequency domain referred to herein as "time-frequency analysis". Because of the nature of the light signal captured by the aquatic biosensors, time-frequency analysis can reveal when and how the frequency of the signal changes. In a preferred embodiment, only three features extracted from the wavelet coefficients are used in the algorithm instead of the entire set of coefficients for signal characterization.

Amplitude statistics and time-frequency analysis according to the invention can be used independently to provide detection results significantly improved as compared to algorithms based on the fluorescence signal signature. However, by combining amplitude statistics and time-frequency analysis, the confidence detection and identification can be improved to an even higher level.

The gas or liquid medium to be monitored or analyzed is generally air in the case of gas and water in the case of liquid. The water can be primary-source drinking water. In a preferred embodiment of the invention, algae is the biosensor used to generate time-dependent biosensor signals such as fluorescence induction curves which are analyzed through extraction of feature vectors to permit classification of different toxic agents in sunlight-exposed primary-source drinking water based on feature vectors. Agents studied included methyl parathion (MPt), potassium cyanide (KCN), Diuron (DCMU), and Paraquat in both the samples of Clinch River (Tennessee) and the samples with lab-grown *Chlamydomonas reinhardtii.*

Biosensors are generally cell-based, and can include genetically modified cells. For example, a bacterium modified with lux genes can be used. In the case of fluorescence induction, algae can be used, either naturally-occurring or genetically modified. Naturally- occurring aquatic algae does not generally require culturing.

Every water source that is exposed to sunlight contains populations of photosynthetic microorganisms (phytoplankton and algae, for example), at concentrations ranging from 10 to as high as 100,000 organisms/ml. Although always present in sunlight-exposed water, these microorganisms are often invisible to the unaided eye. Phytoplankton emits a characteristic fluorescence signal that, if detectable in solutions with low microorganism concentrations, can be utilized as an in situ indicator of chemical and/or biological warfare agents water supplies. Biosensors provide time-dependent biosensor signal while in a gas or liquid medium to be monitored or analyzed for the presence of one or more toxins selected from chemical, biological or radiological agents. Water-soluble toxic chemical and/or biological agents, for example, can include blood agents (cyanide, for example), pesticides (methyl parathion, for example) and herbicides (DCMU, for example), or radionuclide that could pose a threat to primary-source drinking water supplies.

The time-dependent biosensor signal is modified by the toxin as compared to a control signal when the toxin is absent. A variety of signal types can be analyzed using the invention. For example, the signals can be spectroscopic (e.g. fluorescent). Regarding spectroscopic signals, see, e.g., Huang, G.G., Yang, J. 2005 "Development of infrared optical sensor for selective detection of tyrosine in biological fluids", Biosensors and Bioelectronics, 21(3):408-418. Regarding acoustic signals, see, e.g., U.S. Patent 6,486,588 to Doron, et al.

"Acoustic biosensor for monitoring physiological conditions in a body implantation site"; "Acoustic immunosensor for real-time sensing of neurotransmitter GABA", Proceedings of the 25th IEEE Annual International Conference, 4:2998-3000. + Khraiche, M.L., Zhou, A., Muthuswamy, J. 2003, and "Acoustic sensors for monitoring neuronal adhesion in real-time", Proceedings of the 25th IEEE Annual International Conference, 3:2186-2188.). Regarding electrochemical signals, see, e.g., U.S. Patent 6,511,854 to Asanov, et al. "Regenerable biosensor using total internal reflection fluorescence with electrochemical control", and "Development and evaluation of electrochemical glucose enzyme biosensors based on carbon film electrodes" Talanta, 65(2):306-312. + Xu, J.-Z., et al. 2004.

Regarding thermal detection, see e.g.,"Calorimetric biosensors with integrated microfluidic channels. Biosensors and Bioelectronics", 19(12):1733-1743. + Towe, B.C., Guilbeau, E.J. 1996. Regarding magnetic based sensors, see de Oliveira, J.F., et al. 2005 "Magnetic resonance as a technique to magnetic biosensors characterization in Neocapritermes opacus termites" Journal of Magnetism and Magnetic Materials, 292(2): e171-e174. + Chemla, Y.R., et al. 2000, "Ultrasensitive magnetic biosensor for homogeneous immunoassay", Proc. Natl. Acad. Sci. USA, 97(26):14268-72. Regarding surface plasmon resonance (SPR) using enzymes or antibodies see, e.g., U.S. Patent 6,726,881 to Shinoki , et al. "Measurement chip for surface resonance biosensor", U.S. Patent 6,573,107 to Bowen, et al. "Immunochemical detection of an explosive substance in the gas phase through surface plasmon resonance spectroscopy", U.S. Patent 5,313,264 to Ivarsson, et al. "Optical biosensor system".

In the case of air monitoring using algae-based biosensors, the algae generally requires culturing. In this embodiment, air to be analyzed can be drawn through filter paper having algae cultured thereon.

Although the invention is generally hereafter described related to fluorescence induction provided by algal biosensors in water, as noted above, the invention is in no way limited to this specific embodiment.

Feature Extraction of Fluorescence Induction Curves

Classification of different toxic agents in primary-source drinking water through the analysis of fluorescence induction curves is a challenging undertaking. It is difficult to separate different curves by simply looking at the amplitude responses of the curves. Statistical analysis according to the invention can describe, for example, how "fast" the curve reaches the maximum, how "slow" the curve decreases after reaching the maximum. These features are largely related to high-order statistics. In addition, further analyses in other transformation domains (frequency or time-frequency) as described below are also preferably performed in order to provide additional information related to the frequency change over time.

Amplitude Statistics

Amplitude statistics provide statistical measurements of the biosensor signal to be analyzed, such as average fluorescence amplitude over time. The mathematical definition of amplitude statistics up to the fourth order is as follows:

$$\text{mean: } \mu_{amp} = \frac{1}{N}\sum_{i=1}^{N} F(i)$$

standard deviation:

$$\sigma_{amp} = \sqrt{\frac{1}{N}\sum_{i=1}^{N}\left(F(i)-\mu_{amp}\right)^2}$$

$$\text{skewness: } \dot{\gamma}_{amp} = \frac{1}{N}\sum_{i=1}^{N}\left(\frac{F(i)-\mu_{amp}}{\sigma_{amp}}\right)^3$$

$$\text{kurtosis : } \beta_{amp} = \frac{1}{N}\sum_{i=1}^{N}\left(\frac{F(i)-\mu_{amp}}{\sigma_{amp}}\right)^4$$

where, regarding fluorescence, $F(i)$ represents the relative fluorescence at the $i$th time point, and $N$ is the number of time points in the induction curve.

The first-order statistics, the mean, is actually the average of amplitude. The second-order statistics, the *standard deviation,* reflects how different each sample is from the mean. The third-order statistics, the *skewness,* depicts how symmetric the curve is. The fourth order statistics, the *kurtosis*, describes how flat the curve is. The flatter the curve, the less the kurtosis.

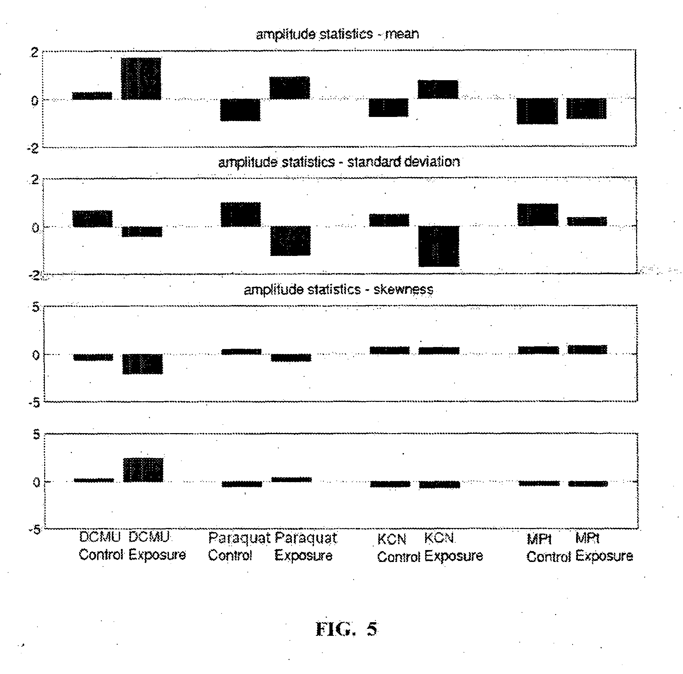

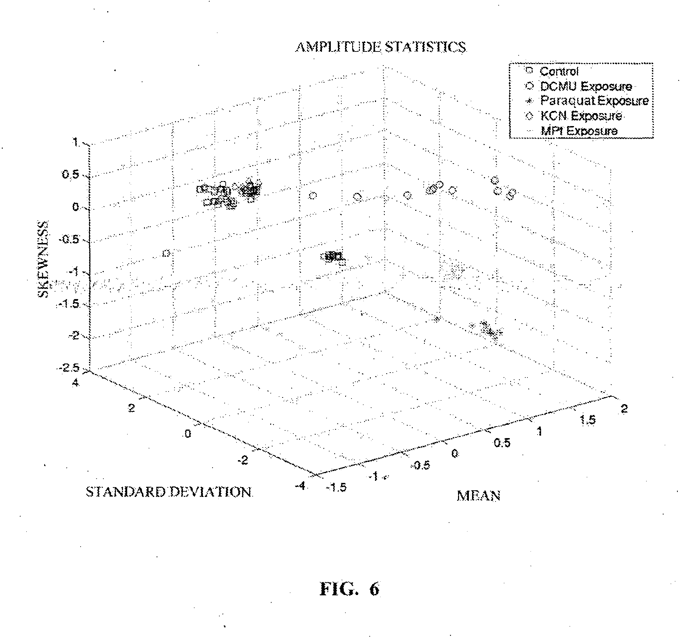

Figure 5 compares the average amplitude statistics generated from the control and exposure signals of each toxic agent class. Figure 6 shows the 3-D plot regarding to the three amplitude statistics features (mean, standard deviation, and skewness). It can be seen from Figure 6 that data from different classes of toxic agents cluster at different positions within the 3D feature space that can be separated relatively easily. This data demonstrates the effectiveness of using amplitude statistics in feature extraction to classify among different toxic chemical agents.

Statistics in Wavelet Coefficients

The wavelet transform is a generalization of the well-known Fourier transform in signal processing. The Fourier transform represents a signal in the frequency domain by decomposing a waveform into sinusoids of different frequencies with different amplitudes (weights) which sum to the original waveform. In another word, the Fourier transform reveals the amount of each frequency component needed to form the original waveform. Although informative, the Fourier transform does not preserve any information concerning the time domain, e.g.,

when and how long in the time domain that a specific frequency component occurs. The lack of time-domain information in the Fourier transform presents a critical problem for the analysis of non-stationary signals which do not maintain the same frequency component throughout the duration of the signal. For example, a stationary signal with four frequency components (e.g. 10Hz, 25Hz, 50Hz, and 100Hz) at all times, and a non-stationary signal with the same four frequency components occurring at different time periods will have the same Fourier transform despite the obvious difference presented in the respective time-domain signals.

Unlike the Fourier transform, time-frequency analysis, such as provided by the wavelet transform, presents a time-frequency representation of the signal. A time-frequency representation of the signal provides time-domain information that a specific spectral component occurs. Since biosensor signals such as the fluorescence induction signals are generally non-stationary, it is beneficial to apply a time-frequency analysis such as the wavelet transform to present both the time-domain and the frequency-domain information.

Different from the Fourier transform that uses the sine and cosine functions as basis functions, wavelet transforms use basic functions that are localized in both time and frequency domains. Wavelet transforms aim at representing the time function in terms of simple, fixed models, which are called wavelets. Wavelets are derived from a single generating function that is called the mother wavelet. The mother wavelet meets the following conditions:

$$\int \psi(t)dt = 0, \quad \psi_{a,b}(t) = \frac{1}{\sqrt{a}}\psi\left(\frac{t-b}{a}\right)$$

where $a$ is the scaling factor and $b$ is the translation factor. The translation and scaling of the mother wavelet will generate a family of functions. The parameter a changes the scale of the wavelet, that is, the greater $|a|$ is, the smaller the frequency. The parameter $b$ controls the translation of the wavelet. In other words, wavelet transforms use narrower windows when the signal frequencies are high and wider windows when the signal frequencies are low. This representation allows the wavelet transform to "enlarge" every high-frequency component, such as the transient in signals. This is one of the main advantages of the wavelet transform over the Fourier transform. The wavelet transform could be categorized into the continuous wavelet transform (CWT) and the discrete wavelet transform (DWT), defined as:

$$CWT_f(a,b) = |a|^{-1/2}\int_{-\infty}^{\infty} f(t)\psi\left(\frac{t-b}{a}\right)dt.$$

$$DWT_f(m,n) = a_0^{-m/2} \int_{-\infty}^{\infty} f(t)\psi(a_0^{-m}t - nb_0)dt$$

Considering the computation complexity of CWT, given sampled signals of finite length, it is generally preferable to use then DWT with the invention. In the DWT the family of wavelets is given as:

$$\psi_{m,n}(t) = a_0^{-m/2}\psi(a_0^{-m}t - nb_0)$$

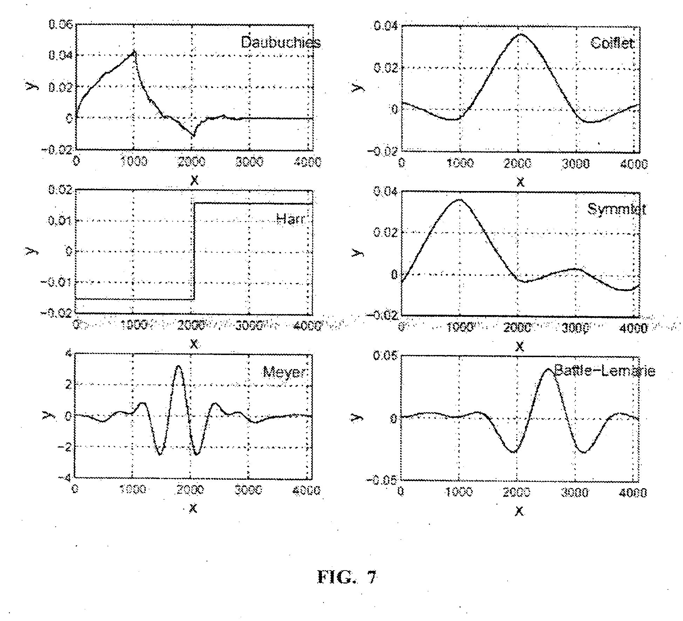

Some examples of some exemplary mother wavelets are shown in Fig. 7, including the Daubechies wavelet, the Coiflet wavelet, the Harr wavelet, the Symmlet wavelet, the Meyer wavelet and the Battle Lemarie wavelet. Among these wavelets, the Daubechies wavelet is popularly used in the engineering field. The parameters of the corresponding Daubechies wavelets are given by sequence $(h_0, h_1, \cdots, h_N)$ that satisfies the following conditions:

$$\sum_{n=0}^{N} h_n = \sqrt{2} \, ,$$

$$\sum_{n=0}^{N} (-1)^n n^k h_n = 0, \text{ for } k = 0,1,2,\cdots,(N-1)/2 \, ,$$

and

$$\sum_{n=0}^{N-2k} h_n h_{n+2k} = 0, \text{ for } k = 1,2,3,\cdots,(N-1)/2 \, .$$

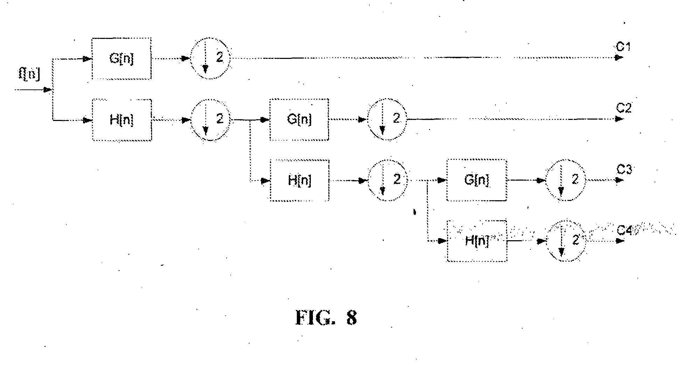

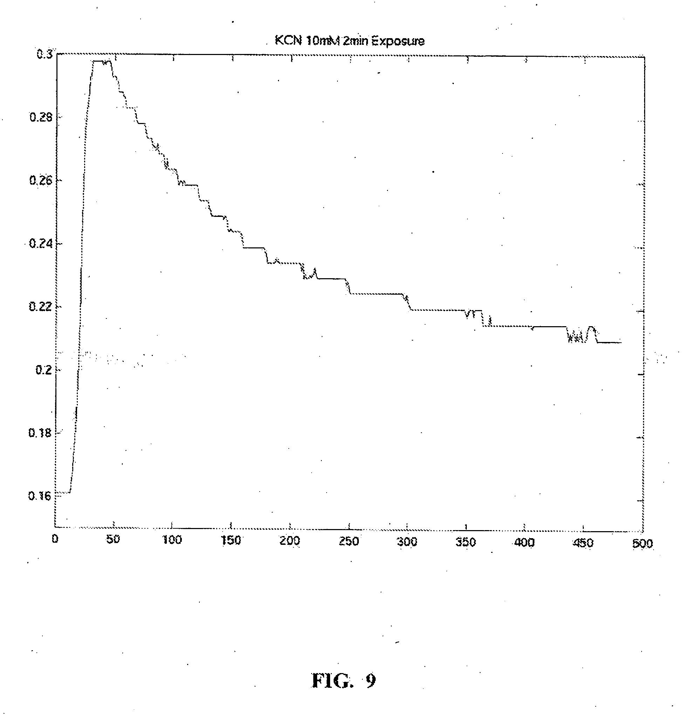

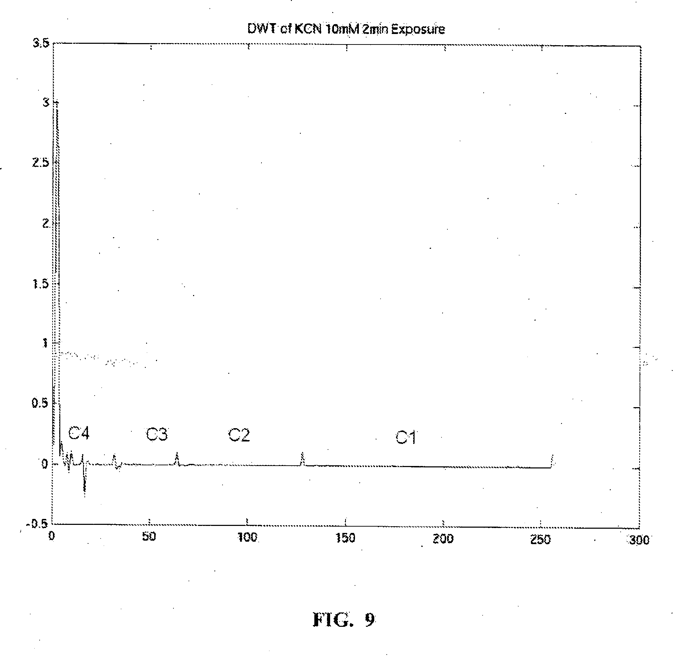

In practice, the forward and inverse wavelet transforms could be implemented using a set of sampling functions, called *digital filter banks* as shown in Fig. 8. The sampled data $f[n]$ is first input in parallel to a low-pass filter (H[n]) and a high-pass filter (G[n]). The outputs from the two filters are down-sampled and thus kept exactly one half of the size of the input signals. After the first stage, the output of the high-pass filter becomes the first-level wavelet coefficients, C1. The second stage uses the output from the low-pass filter of the previous step as the input, which is again sent to H[n] and G[n]. The output of the down-sampled high-pass filter of this stage becomes the second-level wavelet coefficients, C2. The same process continues until only two sample points are left. In another word, the DWT uses filters of different cutoff frequencies to analyze the signal at different scales. The signal is passed through a series of high-pass and low-pass filters to analyze the high frequency and low frequency contents respectively. Figure 8 shows a three-level wavelet transform with four series of coefficients, C1 to C4. Figure 9 shows an example of a fluorescence induction curve exposed to 10 mM KCN and the corresponding 3-level wavelet decomposition.

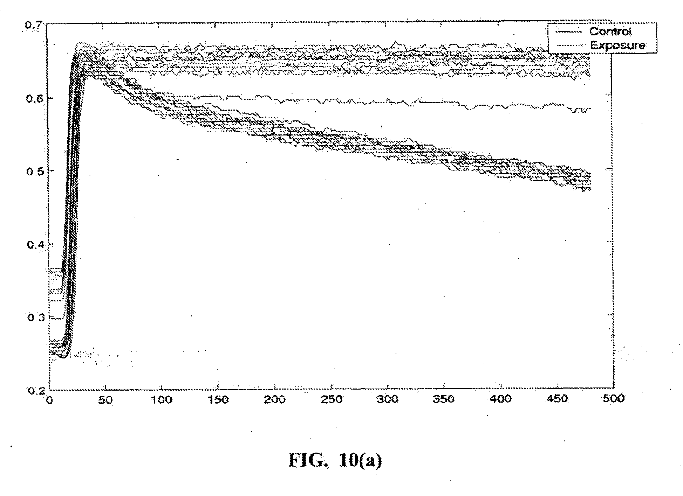

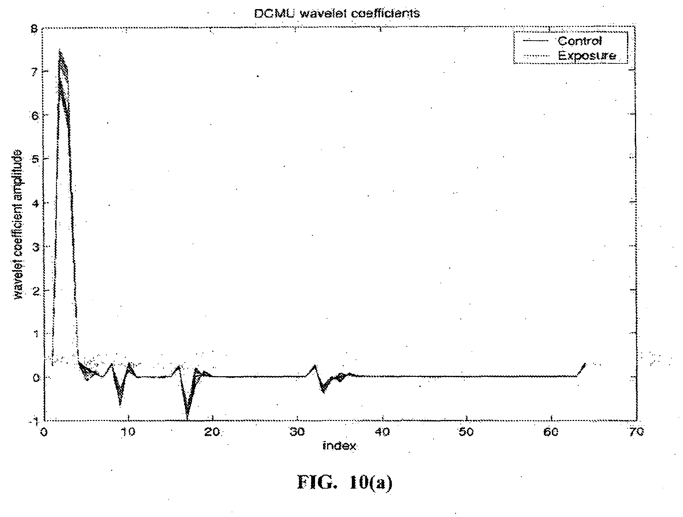

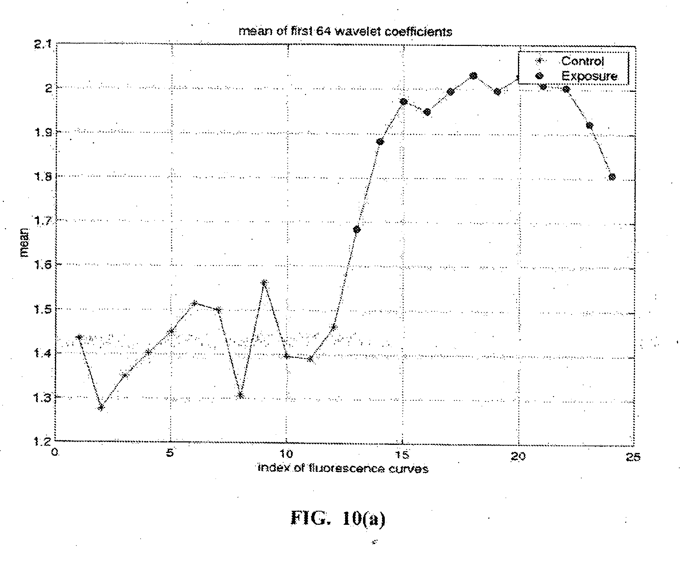

Figure 10(a)-(d) show fluorescence induction curves exposed to four classes of toxic agents and the corresponding wavelet transforms of these signals. In a preferred embodiment, each original induction curve is preprocessed by down-sampling them into signals with 256 discrete samples and then apply the DWT. Suppose the highest frequency component existed in the fluorescence induction signal is $f_{max}$, then in a three-level wavelet decomposition, the C1 wavelet coefficients of 128 samples correspond to spectral components within frequency band $(f_{max} / 2, f_{max})$ ; the C2 coefficients of 64 samples reflect frequency components of band $(f_{max} / 4, f_{max} / 2)$, the C3 coefficients of 32 samples correspond to band $(f_{max} / 8, f_{max} / 4)$, and the C4 coefficients of 32 samples are the low-pass content of band $(0; f_{max} / 4)$. Figure 9 shows one example of the 3-level decomposition.

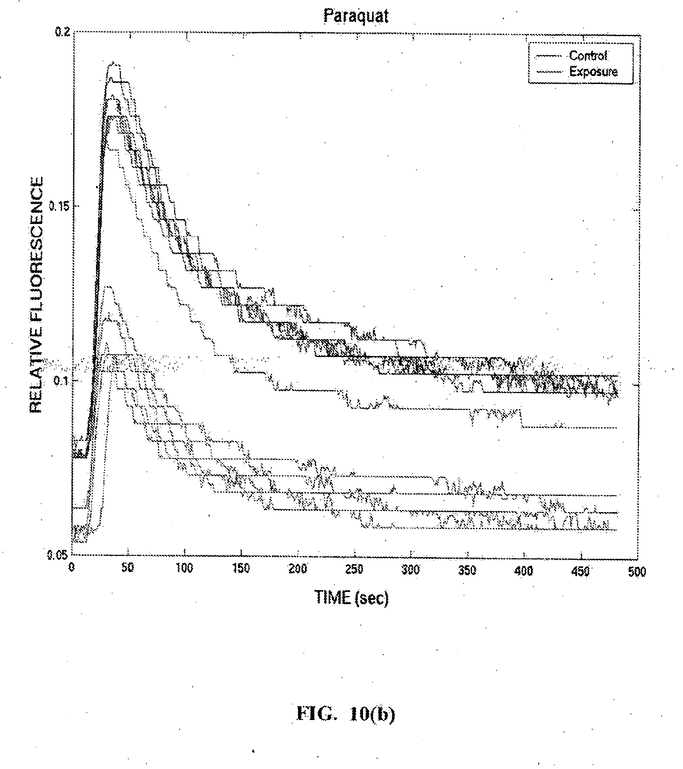

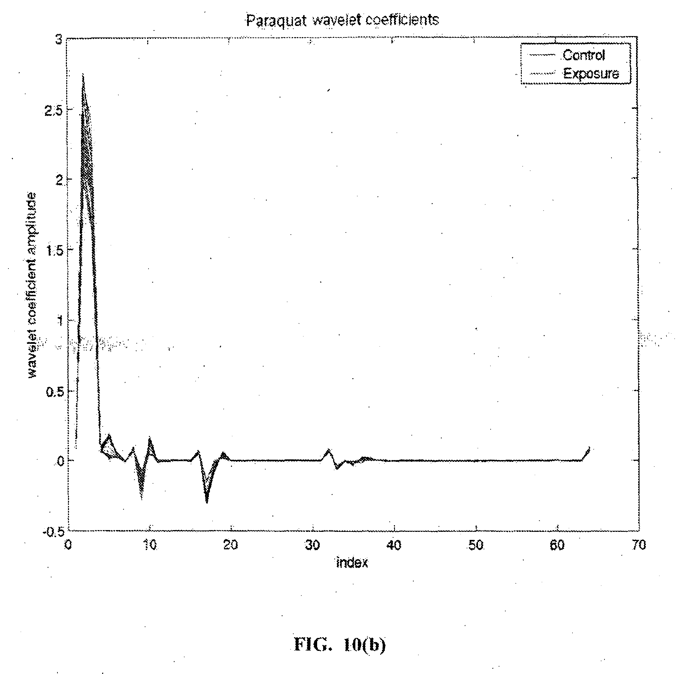

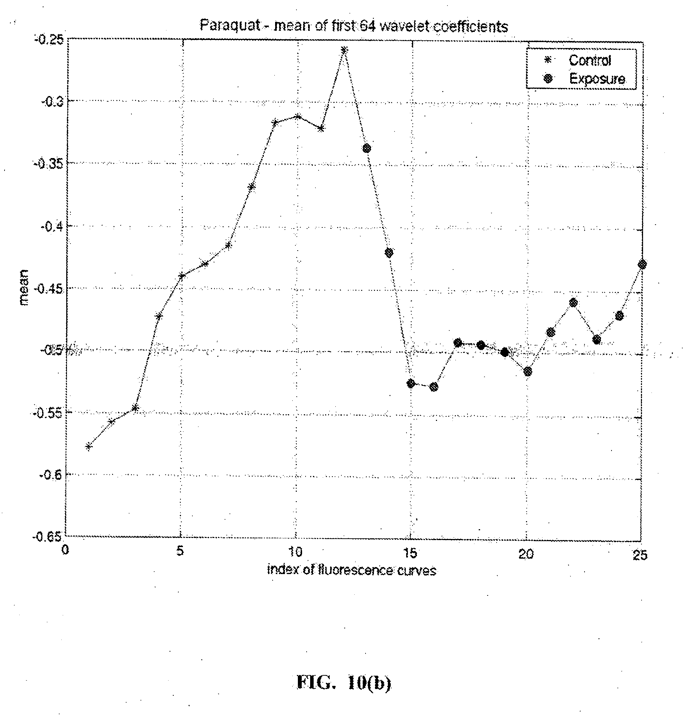

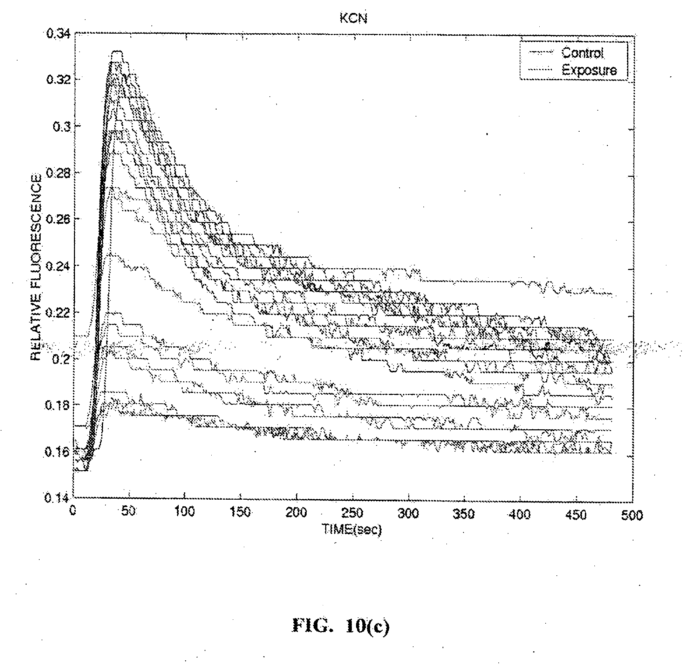

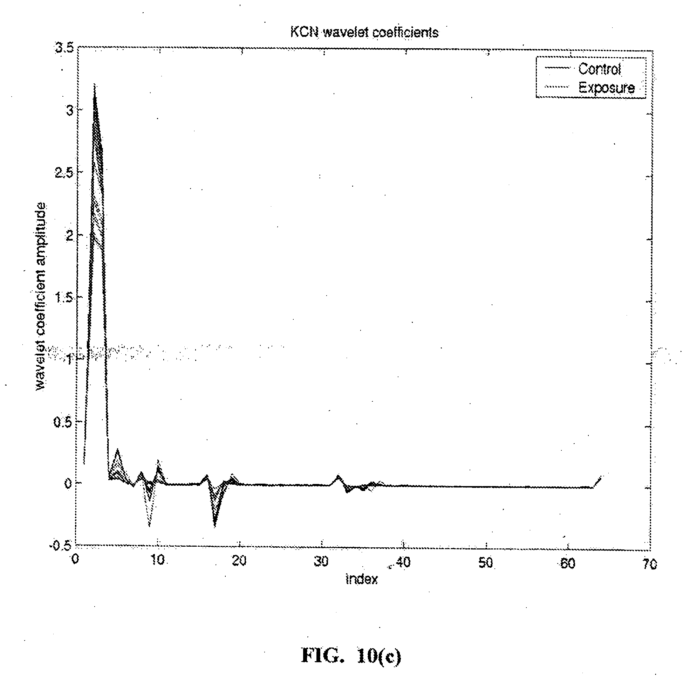

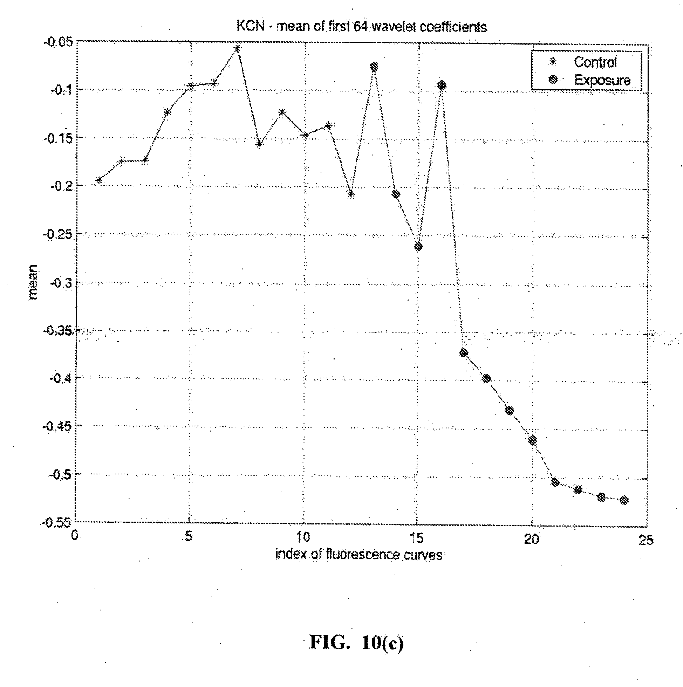

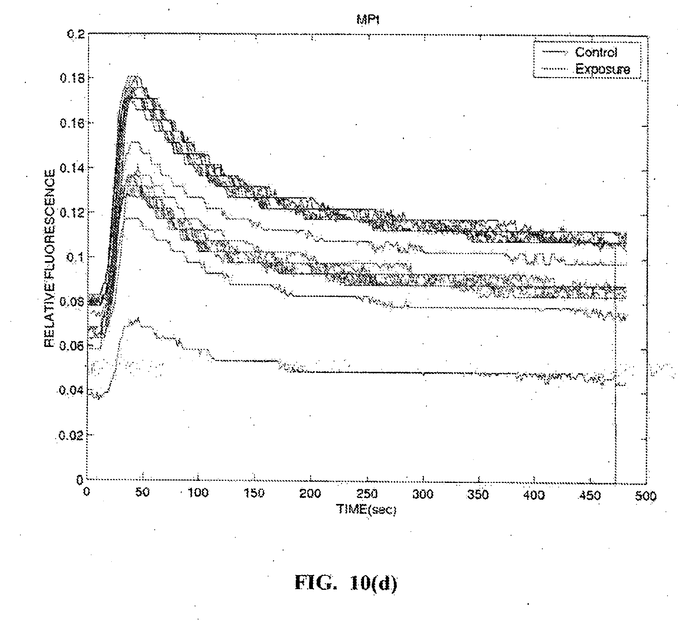

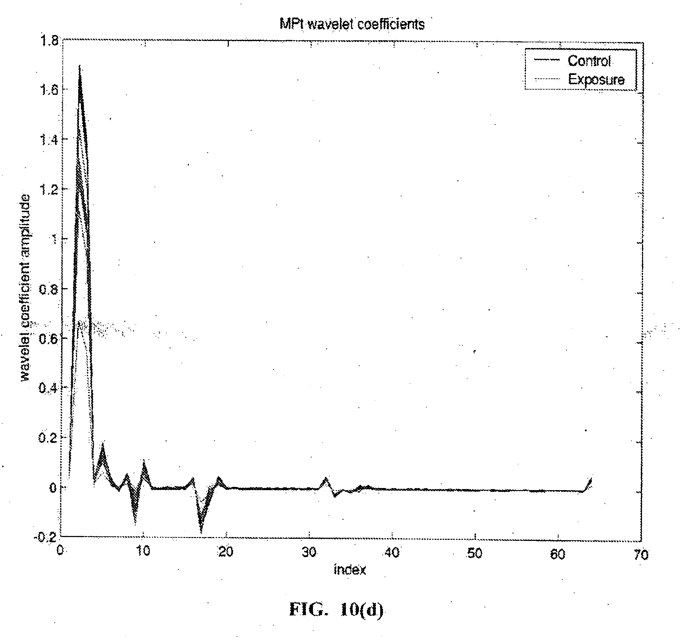

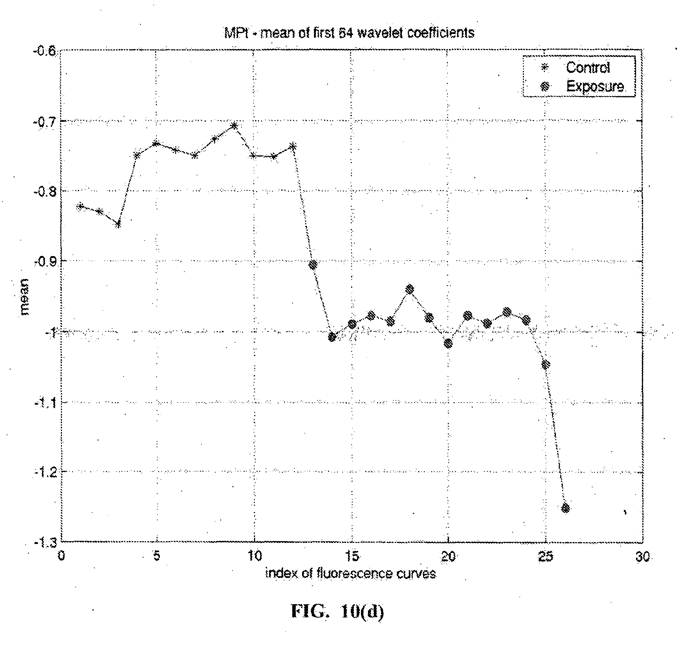

Since the fluorescence induction signals are mainly composed of very-low-frequency components, only the first 64 wavelet coefficients (C4 and C3) that correspond to low-frequency spectral components are considered and shown in Fig. 10.

Existing methods use the wavelet coefficients themselves to serve as a feature set for classification. However, not all the coefficients are necessary. In addition, the use of all generated coefficients would increase the dimensionality of the feature set and bring both computation burden and the "insufficient training sample" problem. Therefore, it is generally preferable to choose to calculate only three statistical features from the first 64 wavelet coefficients: the mean, the variance, and the energy. The mean of the first 64 wavelet coefficients is plotted in Fig. 10 as an example to show the distinction between control and exposure data of different toxic agent classes.

Classifier Design

A supervised classifier is preferably used to differentiate among different toxic agents. Among all existing supervised classification algorithms, a linear discriminant method is preferred, such as the support vector machine (SVM) technique (Duda, R.O., Hart, P.E., Stork, D.G. 2001, Pattern classification; John Wiley & Sons, 2nd edition). The SVM classifier relies on transforming the data to represent patterns in a much higher dimension than the original feature space. With an appropriate nonlinear mapping (which is application specific) to a sufficiently high dimension, data from different categories can be separated by hyperplanes. The optimal hyperplane between each pair of classes is decided by the support vectors that are the most informative training samples for the classification task. Compared to other classification methods, SVM solves the problem of overfitting since the complexity of the classifier is characterized by the number of support vectors instead of the dimensionality of the transformed space. Before applying the classifier, a normalization phase is preferably conducted based on the features extracted from the raw data using algorithms described above.

Toxic substances often appear in primary-source drinking waters because of unintentional industrial activity or potentially deliberate human action, and its real-time detection is of great importance because bioterrorism and environment contamination are on the rise around the globe. Current practices of prevention rely on real-time monitoring. Since the threat of toxins to societies could be substantially mitigated with early detection, the demand for rapid detection of low concentration toxins is expanding quickly for both civilian and military applications to improve homeland security. The invention can detect toxins at very low concentrations, particularly when embodied using the preferred lab-on-a chip cell, thus providing an earlier warning than previously possible. Systems according to the invention will provide a unique advantage to managers of water utility facilities at civilian and military installations for early detection in the event of a contamination event.

<u>EXAMPLES</u>

It should be understood that the Examples described below are provided for illustrative purposes only and do not in any way define the scope of the invention.

In a first experiment, high-efficiency bioparticle concentration was demonstrated using an ACEO cell with an AC bias of less than 3 volts. It was found that about 60 cells *Escherichia coli* were collected into a surface area of 10 $\mu$m x 10 $\mu$m from a suspension of $10^6$ particles/ml (*E. coli* cells were about 100 $\mu$m apart in the solution) within 30 seconds.

In another experiment, *Chlorella vulgaris* (a green alga) was selected as the model bioparticle. To maximize detection signals, thin film metal electrodes with feature sizes of micrometer and nanometer, comparable to those of bioparticles, were used to study electrokinetic capture of bioparticles and their autofluorescence on these electrodes.

Measuring the impedance of detection electrodes at electrical signals suitable for bioparticle trapping, a resolution better than $10^4$ bacteria/ml and impedance differentiation between two types of bioparticles were obtained. Bioparticles attracted to the electrodes reduce the impedance measured between the pair of electrodes. So by comparing the measured impedance reading with a control sample, the difference in impedance will indicate the presence of the bioparticles.

A video file was obtained with the alga *Chlorella vulgaris.* The results obtained demonstrated that electrofocusing can increase local concentration of microalgae by several orders of magnitude in real-time. In the video file obtained, the cells were seen to form a single file at the left electrode. Each electrode was about 70 micrometers ($\mu$m) in diameter.

It is to be understood that while the invention has been described in conjunction with the preferred specific embodiments thereof, that the foregoing description as well as the examples which follow are intended to illustrate and not limit the scope of the invention. Other aspects, advantages and modifications within the scope of the invention will be apparent to those skilled in the art to which the invention pertains.

**Claims**

1. A method of biosensor-based detection of toxins, comprising the steps of:

   concentrating a plurality of photosynthetic organisms in a fluid to be analyzed into a concentrated region using biased AC electro-osmosis; obtaining a measured photosynthetic activity of said photosynthetic organisms in said concentrated region, wherein chemical, biological or radiological agents reduce a nominal photosynthetic activity of said photosynthetic organisms, and determining a presence of at least one of said chemical, biological or radiological agents, or precursors thereof, in said fluid based on said measured photosynthetic activity.

2. The method of claim 1, wherein said plurality of photosynthetic organisms are naturally-occurring, free-living, indigenous organisms in said fluid.

3. The method of claim 1, wherein said photosynthetic activity comprises chlorophyll fluorescence induction.

4. The method of claim 1, wherein a lab-on-a-chip system is used for said concentrating step.

5. The method of claim 1, wherein said fluid is drawn from a source of primary-source drinking water.

6. The method of claim 5, further comprising the step of refreshing a supply of said photosynthetic organisms by drawing a fresh supply of said drinking water and repeating said method.

7. The method of claim 1, wherein said determining step further comprises the steps of:

   providing at least one time-dependent control signal generated by said photosynthetic organism in said fluid; obtaining a time-dependent biosensor signal from said biosensor in said fluid for the presence of one or more of said agents;
processing said time-dependent biosensor signal to obtain a plurality of feature vectors using at least one of amplitude statistics and a time-frequency analysis, and
determining said presence of at least one of said chemical, biological or radiological agents, or precursors thereof, from said feature vectors based on reference to said control signal.

**8.** The method of claim 7, wherein said time-frequency analysis comprises wavelet coefficient analysis.

**9.** The method of claim 7, wherein both said amplitude statistics and time-frequency analysis are used in said processing step.

**10.** The method of claim 7, further comprising the step of identifying which of said agents are present in said fluid.

**11.** The method of claim 10, wherein a linear discriminant method is used for said identifying step.

**12.** The method of claim 11, wherein said linear discriminant method comprises support vector machine (SVM) classification.

**13.** The method of claim 1, wherein a DC bias for said biased ACEO is from 1 to 10 volts.

**14.** A water quality analyzer, comprising:

a biased AC electro-osmosis (ACEO) cell for receiving a fluid to be analyzed having a plurality photosynthetic organisms therein and concentrating said plurality photosynthetic organisms into at least one concentrated region, and
a photodetector for obtaining a measured photosynthetic activity of said plurality of photosynthetic organisms in said concentrated region, wherein chemical, biological or radiological agents reduce a nominal photosynthetic activity of said photosynthetic organisms, and
an electronics package for analyzing measured photosynthetic activity to indicate a presence of said chemical, biological or radiological agents in said fluid.

**15.** The sensor of claim 14, wherein said ACEO cell comprises a lab-on-a-chip device.

**16.** The sensor of claim 15, wherein said lab-on-a-chip device includes at least one electronic device on said chip.

**17.** The sensor of claim 14, further comprising structure for communicating said measured photosynthetic activity or said measured photosynthetic activity after analysis by said electronics package to one or more remote sites.

**Patentansprüche**

**1.** Ein Verfahren zum Nachweis von Giftstoffen, basierend auf einem Biosensor, das folgende Schritte aufweist:

Konzentration einer Mehrzahl fotosynthetischer Organismen in einer Flüssigkeit, um sie in einen konzentrierten Bereich unter Verwendung von polarisierter Wechselstromelektroosmose zu zergliedern;
Erreichen einer gemessenen fotosynthetischen Aktivität der fotosynthetischen Organismen in der konzentrierten Region, **dadurch gekennzeichnet, dass** chemische, biologische oder radiologische Wirkstoffe die nominale fotosynthetische Aktivität der fotosynthetischen Organismen reduzieren, und
Ermittlung des Vorhandenseins von mindestens einem der besagten chemischen, biologischen oder radiologischen Wirkstoffe oder Vorläufer von diesen in der Flüssigkeit, basierend auf der gemessenen fotosynthetischen Aktivität.

**2.** Das Verfahren nach Anspruch 1, **dadurch gekennzeichnet, dass** die Mehrzahl der fotosynthetischen Organismen natürlich vorkommende, freilebende, indigene Organismen in der Flüssigkeit sind.

**3.** Das Verfahren nach Anspruch 1, **dadurch gekennzeichnet, dass** die fotosynthetische Tätigkeit Chlorophyllfluoreszenz-Induktion aufweist.

**4.** Das Verfahren nach Anspruch 1, **dadurch gekennzeichnet, dass** ein Lab-on-a-Chip-System für den Konzentrationsschritt verwendet wird.

**5.** Das Verfahren nach Anspruch 1, **dadurch gekennzeichnet, dass** die Flüssigkeit aus einer Primärtrinkwasserquelle entnommen wird.

**6.** Das Verfahren nach Anspruch 5, das weiterhin den Schritt der Auffrischung der Versorgung mit den fotosynthetischen Organismen aufweist, indem eine frische Versorgung mit dem Trinkwasser vorgenommen und das Verfahren wiederholt wird.

**7.** Das Verfahren nach Anspruch 1, **dadurch gekennzeichnet, dass** der Ermittlungsschritt weiterhin folgende Schritte aufweist:

Schaffung von mindestens einem zeitabhängigen Steuersignal, das durch die fotosynthetischen Organismen in der Flüssigkeit generiert wird;

Erwirken eines zeitabhängigen Biosensorsignals aus dem Biosensor in der Flüssigkeit für das Vorhandensein von einem oder mehreren Wirkstoffe;

Verarbeitung des zeitabhängigen Biosensorsignals, um eine Mehrzahl der Eigenschaftsvektoren unter Verwendung von wenigstens einer Amplitudenstatistik und einer Zeit-Frequenz-Analyse zu erhalten, und

Ermittlung des Vorhandenseins von mindestens einem der chemischen, biologischen oder radiologischen

Wirkstoffe oder Vorläufer von diesen aus den Eigenschaftsvektoren, basierend auf der Referenz des Steuersignals.

8. Das Verfahren nach Anspruch 7, **dadurch gekennzeichnet, dass** die Zeit-Frequenz-Analyse eine Wavelet-Koeffizienten-Analyse aufweist.

9. Das Verfahren nach Anspruch 7, **dadurch gekennzeichnet, dass** sowohl Amplitudenstatistiken und Zeit-Frequenz Analyse in dem Verarbeitungsschritt eingesetzt werden.

10. Das Verfahren nach Anspruch 7, das weiterhin den Schritt der Identifizierung aufweist, welche der Wirkstoffe in der Flüssigkeit vorhanden sind.

11. Das Verfahren nach Anspruch 10, **dadurch gekennzeichnet, dass** ein lineares Diskriminanzverfahren für die Identifizierungsschritte angewendet wird.

12. Das Verfahren nach Anspruch 11, **dadurch gekennzeichnet, dass** das lineare Diskriminanzverfahren eine Support Vector Machine (SVM)-Klassifizierung aufweist.

13. Das Verfahren nach Anspruch 1, **dadurch gekennzeichnet, dass** die Gleichstrompolarisation für die polarisierte ACEO zwischen 1 und 10 Volt beträgt.

14. Ein Wasserqualitätsanalysegerät, das Folgendes aufweist:

eine polarisierte Wechselstromelektroosmose (ACEO) für das Erhalten einer zu analysierenden Flüssigkeit, die darin eine Mehrzahl von photosynthetischen Organismen enthält und die Konzentration der Mehrzahl der fotosynthetischen Organismen in wenigstens einen konzentrierten Bereich und

einen Fotodetektor für das Erhalten einer gemessenen fotosynthetischen Tätigkeit der Mehrzahl von fotosynthetischen Organismen in der konzentrierten Region, **dadurch gekennzeichnet, dass** chemische, biologische oder radiologische Wirkstoffe die nominale fotosynthetische Aktivität der fotosynthetischen Organismen verringern, und

ein Elektronikpaket für das Analysieren der gemessenen fotosynthetischen Aktivität, um das Vorhandensein von chemischen, biologischen oder radiologischen Wirkstoffen in der Flüssigkeit anzuzeigen,

15. Der Sensor nach Anspruch 14, **dadurch gekennzeichnet, dass** die ACEO-Zelle eine Lab-on-a-Chip-Vorrichtung aufweist.

16. Der Sensor nach Anspruch 15, **dadurch gekennzeichnet, dass** die Lab-ona-Chip-Vorrichtung mindestens eine elektronische Vorrichtung auf dem Chip beinhaltet.

17. Der Sensor nach Anspruch 14, der weiterhin eine Struktur für die Übertragung der gemessenen fotosynthetischen Aktivität oder der gemessenen fotosynthetischen Aktivität nach der Analyse durch das Elektronikpaket zu einer oder mehreren entfernten Stellen aufweist.

## Revendications

1. Procédé de détection de toxines par biocapteur, comprenant les étapes suivantes :

la concentration d'une pluralité d'organismes photosynthétiques dans un fluide à analyser en une région concentrée par électro-osmose à polarisation en courant alternatif ;
l'obtention d'une activité photosynthétique mesurée desdits organismes photosynthétiques dans ladite région concentrée, dans laquelle des agents chimiques, biologiques ou radiologiques réduisent une activité photosynthétique nominale desdits organismes photosynthétiques, et
la détermination d'une présence d'au moins un desdits agents chimiques, biologiques ou radiologiques, ou de leurs précurseurs, dans ledit fluide sur la base de ladite activité photosynthétique mesurée.

2. Procédé selon la revendication 1, dans lequel ladite pluralité d'organismes photosynthétiques est une pluralité d'organismes indigènes d'origine naturelle en développement libre dans ledit fluide.

3. Procédé selon la revendication 1, dans lequel ladite activité photosynthétique comprend l'induction de la fluorescence de la chlorophylle.

**4.** Procédé selon la revendication 1, dans lequel un système à laboratoire sur puce est utilisé pour ladite étape de concentration.

**5.** Procédé selon la revendication 1, dans lequel ledit fluide provient d'une source primaire d'eau potable.

**6.** Procédé selon la revendication 5, comprenant en outre l'étape de renouvellement desdits organismes photosynthétiques par le prélèvement d'un nouvel approvisionnement de ladite eau potable et par la répétition dudit procédé.

**7.** Procédé selon la revendication 1, dans lequel ladite étape de détermination comprend en outre les étapes suivantes :

la fourniture d'au moins un signal témoin dépendant du temps généré par lesdits organismes photosynthétiques dans ledit fluide ;
l'obtention d'un signal de biocapteur dépendant du temps provenant dudit biocapteur dans ledit fluide pour la présence d'un ou de plusieurs desdits agents ;
le traitement dudit signal de biocapteur dépendant du temps pour obtenir une pluralité de vecteurs d'attribut au moyen d'au moins une analyse choisie parmi une analyse statistique d'amplitude et une analyse de temps-fréquence, et
la détermination de ladite présence d'au moins un desdits agents chimiques, biologiques ou radiologiques, ou de leurs précurseurs, à partir desdits vecteurs d'attribut sur la base de la référence dudit signal témoin.

**8.** Procédé selon la revendication 7, dans lequel ladite analyse de temps-fréquence comprend une analyse de coefficient d'ondelette.

**9.** Procédé selon la revendication 7, dans lequel ladite analyse statistique d'amplitude et ladite analyse de temps-fréquence sont utilisées dans ladite étape de traitement.

**10.** Procédé selon la revendication 7, comprenant en outre l'étape consistant à identifier lesquels desdits agents sont présents dans ledit fluide.

**11.** Procédé selon la revendication 10, dans lequel un procédé discriminant linéaire est utilisé pour ladite étape d'identification.

**12.** Procédé selon la revendication 11, dans lequel ledit procédé discriminant linéaire comprend un classement par machine à vecteurs de support (SVM).

**13.** Procédé selon la revendication 1, dans lequel une polarisation en courant continu pour ladite électro-osmose à polarisation en courant alternatif (ACEO) est de 1 à 10 volts.

**14.** Dispositif d'analyse de la qualité de l'eau, comprenant :

une cellule d'électro-osmose à polarisation en courant alternatif (ACEO) pour recevoir un fluide à analyser contenant une pluralité d'organismes photosynthétiques et concentrer ladite pluralité d'organismes photosynthétiques en au moins une région concentrée, et
un photodétecteur pour obtenir une activité photosynthétique mesurée de ladite pluralité d'organismes photosynthétiques dans ladite région concentrée, dans lequel des agents chimiques, biologiques ou radiologiques réduisent une activité photosynthétique nominale desdits organismes photosynthétiques, et
un assemblage électronique pour analyser l'activité photosynthétique mesurée afin d'indiquer une présence desdits agents chimiques, biologiques ou radiologiques dans ledit fluide.

**15.** Capteur selon la revendication 14, dans lequel ladite cellule d'ACEO comprend un dispositif à laboratoire sur puce.

**16.** Capteur selon la revendication 15, dans lequel ledit dispositif à laboratoire sur puce comprend au moins un dispositif électronique sur ladite puce.

**17.** Capteur selon la revendication 14, comprenant en outre une structure servant à communiquer ladite activité photosynthétique mesurée ou ladite activité photosynthétique mesurée après analyse par ledit assemblage électronique à un ou plusieurs sites éloignés.

**FIG. 1**

FIG. 2

EP 1 957 663 B1

**FIG. 3**

**FIG. 4**
(PRIOR ART)

amplitude statistics - mean

amplitude statistics - standard deviation

amplitude statistics - skewness

DCMU DCMU
Control Exposure

Paraquat Paraquat
Control Exposure

KCN KCN
Control Exposure

MPt MPt
Control Exposure

FIG. 5

FIG. 6

**FIG. 7**

FIG. 8

FIG. 9

DWT of KCN 10mM 2min Exposure

FIG. 9

FIG. 10(a)

FIG. 10(a)

FIG. 10(a)

FIG. 10(b)

FIG. 10(b)

FIG. 10(b)

FIG. 10(c)

FIG. 10(c)

**FIG. 10(c)**

31

FIG. 10(d)

FIG. 10(d)

FIG. 10(d)

## REFERENCES CITED IN THE DESCRIPTION

### Patent documents cited in the description

- US 6569384 B, Greenbaum
- US 6486588 B, Doron
- US 6511854 B, Asanov
- US 6726881 B, Shinoki
- US 6573107 B, Bowen
- US 5313264 A, Ivarsson

### Non-patent literature cited in the description

- **Bhatt, K.H. ; Grego, S. ; Velev, O.D.** An AC electrokinetic technique for collection and concentration of particles and cells on patterned electrodes. *Langmuir,* 2005, vol. 21 (14), 6603-6612
- **Huang, G.G. ; Yang, J.** Development of infrared optical sensor for selective detection of tyrosine in biological fluids. *Biosensors and Bioelectronics,* 2005, vol. 21 (3), 408-418
- **Khraiche, M.L. ; Zhou, A. ; Muthuswamy, J.** Acoustic immunosensor for real-time sensing of neurotransmitter GABA. *Proceedings of the 25th IEEE Annual International Conference,* 2003, vol. 4, 2998-3000
- Acoustic sensors for monitoring neuronal adhesion in real-time. *Proceedings of the 25th IEEE Annual International Conference,* vol. 3, 2186-2188
- **Xu, J.-Z. et al.** Development and evaluation of electrochemical glucose enzyme biosensors based on carbon film electrodes. *Talanta,* 2004, vol. 65 (2), 306-312
- **Towe, B.C. ; Guilbeau, E.J.** Calorimetric biosensors with integrated microfluidic channels. *Biosensors and Bioelectronics,* 1996, vol. 19 (12), 1733-1743
- **de Oliveira, J.F. et al.** Magnetic resonance as a technique to magnetic biosensors characterization in Neocapritermes opacus termites. *Journal of Magnetism and Magnetic Materials,* 2005, vol. 292 (2), e171-e174
- **Chemla, Y.R. et al.** Ultrasensitive magnetic biosensor for homogeneous immunoassay. *Proc. Natl. Acad. Sci. USA,* 2000, vol. 97 (26), 14268-72
- **Duda, R.O. ; Hart, P.E. ; Stork, D.G.** Pattern classification. John Wiley & Sons, 2001